(19) Europäisches Patentamt
European Patent Office
Office européen des brevets

(11) **EP 1 314 416 B1**

(12) **EUROPEAN PATENT SPECIFICATION**

(45) Date of publication and mention
of the grant of the patent:
**24.10.2007 Bulletin 2007/43**

(51) Int Cl.:
*A61K 8/73* (2006.01)    *A61K 8/02* (2006.01)
*A61Q 19/00* (2006.01)

(21) Application number: **01963409.6**

(22) Date of filing: **31.08.2001**

(86) International application number:
**PCT/JP2001/007539**

(87) International publication number:
**WO 2002/017860 (07.03.2002 Gazette 2002/10)**

(54) **WHIPPED O/W EMULSION COSMETIC AND PROCESSES FOR PRODUCING THE SAME**

GESCHLAGENE KOSMETISCHE O/W-EMULSION UND VERFAHREN ZU DEREN HERSTELLUNG

PRODUIT COSMETIQUE EN EMULSION O/W FOUETTEE ET PROCEDE DE PRODUCTION

(84) Designated Contracting States:
**DE FR GB IT**

(30) Priority: **31.08.2000 JP 2000262977**
**31.08.2000 JP 2000262978**

(43) Date of publication of application:
**28.05.2003 Bulletin 2003/22**

(73) Proprietor: **Shiseido Co., Ltd.**
**Tokyo 104-8010 (JP)**

(72) Inventors:
• **SATO, Tomoko,**
**c/o Shiseido Research Center**
**Yokohama-shi,**
**Kanagawa 224-8558 (JP)**
• **MATSUZAKI, Fumiaki,**
**c/o Shiseido Research Center**
**Yokohama-shi,**
**Kanagawa 224-8558 (JP)**
• **YANAKI, Toshio,**
**c/o Shiseido Research Center**
**Yokohama-shi,**
**Kanagawa 224-8558 (JP)**

(74) Representative: **Gudat, Axel et al**
**Lippert, Stachow & Partner**
**Frankenforster Strasse 135-137**
**51427 Bergisch Gladbach (DE)**

(56) References cited:
**EP-A- 0 911 017**      **JP-A- 56 079 613**
**JP-A- 2000 355 517**      **US-A- 3 944 680**

• **DATABASE WPI Section Ch, Week 199223**
**Derwent Publications Ltd., London, GB; Class**
**D21, AN 1992-189209 XP002374009 & JP 04**
**124125 A (NONOGAWA SHOJI YG) 24 April 1992**
**(1992-04-24)**

**Description**

RELATED APPLICATIONS

This application claims the priorities based on Japanese Patent Application No. 2000-262977 filed on August 31, 2000 and Japanese Patent Application No. 2000-262978 filed on August 31, 2000.

BACKGROUND OF THE INVENTION

[FIELD OF THE INVENTION]

The present invention relates to a whipped O/W type emulsion cosmetic and a process for producing the same, in particular, to improvement in the temperature stability, the time course stability and the usability of a whipped O/W type emulsion cosmetic.

[DESCRIPTION OF THE PRIOR ART]

Preparations containing bubbles have the soft texture as compared with preparations containing no bubble and, in particular, in the food field, there are many products containing bubbles such as ice cream, fresh cream and Bavarian cream. Also in the cosmetic field, there are some products utilizing the soft feeling of bubbles obtained by filling in an exclusively used container and discharging from the container to form bubbles, cleansing cosmetic producing bubbles in addition to aerosol products. However, there is scarcely an example of a cosmetic filled in a container while containing bubbles.

When the properties of bubbles are utilized in cosmetics, the aforementioned container for producing bubbles in cosmetic is expensive and, moreover, aerosols need troublesome operation such as removal of air in a container upon waste. For that reason, there has been desired the development of cosmetics filled in a container while containing bubbles as well as in foods. In cosmetics, when filled while containing bubbles, it is necessary that the stability of the bubbles is better. In cosmetics, as the techniques for the retaining the stability of bubbles, there is the formulation for incorporating wax having a high melting point as in JP-A 56-79613, but it is difficult to retain the form while containing bubbles for a long period of time.

Additionally, it is impossible to apply the techniques in the food field to cosmetics as they are. The mechanisms by which the stability of bubbles in foods is retained are different each. For example, ice creams retain a structure by freezing, and fresh cream and margarine retain a structure by forming a structure of lipid sphere whereby, the stability of bubbles is retained. In these food formulations, when taken in a mouth, most of bases are melt at around 30°C and are easily swallowable and, thus, those formulations can not retain a structure at 40°C that is in a guaranteed temperature range for cosmetics. On the other hand, since Bavarian cream and mousse are gelatinized using agar or gelatin while containing bubbles, the stability of bubbles is retained for a relatively long period of time even at a temperature of 40°C or higher. However, since such the preparations have a small amount of an oil which destructs a gel structure, and form a hard gel, they can be bitten with teeth but it is difficult to coat on the skin by scooping with a finger and, thus, they can not be applied as a cosmetic formulation.

Examples of a cosmetic containing agar, gelatin or the like as a polymer material forming a gel having no fluidity which retains the foam stability as described above, include those disclosed, for example, in JP-A 9-249522 and JP-A 11-209262. However, in these cases, a polysaccharide is used mainly as a thickening agent, and the effects of stabilizing the bubbles by the inclusion of much bubbles are not described therein.

Like this, there has been desired the development of a whipped cosmetic which is filled in a container while containing bubbles, has the better foam stability in a wide temperature range for a long period of time, and is excellent in the usability such as scooping with a finger.

BRIEF SUMMARY OF THE INVENTION

In view of the aforementioned problems, the present inventors intensively studied and, as a result, found that a whipped O/W type emulsion cosmetic having the better long-term foam stability and overrun of around 10-300% can be obtained by mixing an O/W type emulsion and a polymer for forming a gel having no fluidity in the state where a gelatinizing agent is dissolved in water, before or after the O/W type emulsion is whipped, and filling it in a container, which resulted in completion of the present invention.

That is, the first subject of present invention is a whipped O/W type emulsion cosmetic characterized in that a gel-forming amount of a polymer for forming a gel having no fluidity is contained in an aqueous phase.

In the cosmetic, it is suitable that the polymer for forming a gel having no fluidity is 1, 2 or more kinds selected from agar, gelatin, sodium alginate, carrageenan, gellan gum, glucomannan and curdlan.

In the cosmetic, it is suitable that an amount of a polymer for forming a gel having no fluidity to be incorporated is 0.1-13% by mass in an aqueous phase.

In the cosmetic, it is suitable that the cosmetic contains a higher fatty acid soap.

In the cosmetic, it is suitable that an amount of a higher fatty acid soap is 1-20% by mass in the whole cosmetic.

In the cosmetic, it is suitable that an amount of an oil to be incorporated is 1-30% by mass in the whole cosmetic.

In the cosmetic, it is suitable that the cosmetic contains a cationic polymer.

In the cosmetic, it is suitable that an amount of a cationic polymer to be incorporated is 0.01-5% by mass in the whole cosmetic.

In the cosmetic, it is suitable that a melting point of a polymer material forming a gel having no fluidity is 40˚C or higher.

In the cosmetic, it is suitable that the cosmetic contains a solid oil which is a solid at a normal temperature and a nonionic surfactant as an emulsifier.

In the cosmetic, it is suitable that an amount of a solid oil to be incorporated is 20-100% by mass relative to a total amount of an oil.

In the cosmetic, it is suitable that the cosmetic contains casein and/or lecithin.

In the cosmetic, it is suitable that an amount of a nonionic surfactant, casein and lecithin to be incorporated is 0.1-15% by mass in the whole cosmetic.

The second subject of the present invention is a process for producing a whipped O/W type emulsion cosmetic, which comprises whipping an O/W type emulsion containing a polymer for forming a gel having no fluidity, and filling it while being whipped.

In addition, the present invention is a process for producing a whipped O/W type emulsion cosmetic, which comprises whipping an O/W type emulsion containing no polymer for forming a gel having no fluidity, mixing a whipped base with a polymer for forming a gel having no fluidity, and filling it.

## DETAILED DESCRIPTION OF THE INVENTION

Examples of the polymer for forming a gel having no fluidity used in the present invention include agar, gelatin, sodium alginate, carrageenan, gellan gum, glucomannan, curdlan and the like.

It is preferable that an amount of the polymer to be incorporated is 0.1-13% by mass, in particular, 1-7% by mass in an aqueous phase in the cosmetic. When the amount is less than 0.1% by mass, it is insufficient for retaining foams in some cases. When the amount exceeds 13% by mass, the cosmetic is too hard to scoop with a finger in some cases.

A. Whipped O/W type emulsion cosmetic containing a higher fatty acid soap

Example of the higher fatty acid soap used in the present invention include sodium and potassium salts of lauric acid, myristic acid, palmitic acid, stearic acid, behenic acid, isostearic acid, oleic acid, linolenic acid, linoleic acid, linolenic acid and oxystearic acid.

It is preferable that an amount of a fatty acid soap to be incorporated is 1-20% by mass, in particular, 5-15% by mass in the whole cosmetic. When the amount is less than 1% by mass, the cosmetic is not sufficiently whipped in some cases. When the amount exceeds 20% by mass, a base becomes hard and the rough feeling in use is generated in some cases.

Examples of the cationic polymer used in the present invention include polyoctanium, polydimethylmethylenepiperidinium chloride, polycoat, cationated hydroxycellulose, methacryloyloxyethylcarboxybetaine methacrylic acid alkyl ester copolymer, polyacrylate and the like.

An amount of the cationic polymer to be incorporated is 0.01-5% by mass, preferably 0.05-3% by mass in the whole cosmetic. When the amount exceeds 5% by mass, the base is felt the stickiness in some cases.

An oil to be incorporated in an O/W type emulsion in the present invention may be any of a liquid oil, a solid oil and a semi-solid oil. Alternatively, a water-slightly soluble substance may be incorporated in an oil phase. By incorporation of an oil, a whipped O/W type emulsion containing a gelling agent does not become too hard and can retain the sufficient softness for scooping with a finger.

Examples of the oil include liquid fats and oils such as avocado oil, camellia oil, turtle oil, macadamia nut oil, corn oil, mink oil, olive oil, rapeseed oil, yolk oil, sesame oil, pearshic oil, wheat germ oil, sasanqua oil, castor oil, linseed oil, safflower oil, cotton seed oil, evening primrose oil, perilla oil, soybean oil, peanut oil, tea seed oil, kaya oil, rice bran oil, Chinese paulownia oil, Japanese paulownia oil, jojoba oil, germ oil, triglycerin, glycerin trioctanoate, glycerin triisopalmitate and the like, solid fats and oils such as cacao butter, coconut oil, horseflesh tallow, hydrogenated coconut oil, palm oil, beef tallow, ram tallow, hardened beef tallow, palm kernel oil, lard tallow, beef bone tallow, Japan wax kernel oil, hardened oil, beef leg tallow, Japan wax, hydrogenated castor oil and the like, waxes such as beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hardened lanolin, shellac wax, POE lanolin alcohol ether, POE lanolin alcohol acetate, lanolin fatty acid polyethylene glycol, POE hydrogenated lanolin alcohol ether and the like, hydrocarbons such as liquid paraffin, ozocerite, squalene, pristane, paraffin, ceresine, squalane, vaseline, microcrystalline wax and the like, and synthetic esters such as isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearylate, ethyleneglycol di-2-ethylhexylate, dipentaerythritol fatty acid ester, N-alkylglycol monoisostearate, neopentylglycol dicapriate, diisostearyl malate, glycerin di-2-heptylundecanoate, trimethylolpropane tri-2-ethylhexylate, trimethylolpropane triisostearate, pentaerythritol tetra-2-ethylhexylate, glycerin tri-2-ethylhexylate, trimethylolpropane triisostearate, cetyl 2-ethylhexanote, 2-ethylhexyl palmitate, glycerin trimyristate, glyceride tri-2-heptylundecanoate, castor oil fatty acid methyl ester, oleic acid oil, cetostearyl alcohol, acetoglyceride, 2-heptylundecyl palmitate, diisopropyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succiniate, ethyl acetate, butyl acetate, amyl acetate, triethyl citrate and the like. Vitamins such as vitamin A and derivatives thereof, vitamin D and derivatives thereof, vitamin E and derivatives thereof, vitamin K and derivatives thereof and the like, sterols, natural and synthetic perfumes and the like may be incorporated. Inter alia, polar oils such as 2-octyldodecanol and the like are suitably used.

In addition to these oily ingredients, a small amount of a silicone oil such as straight polysiloxane and the like such as dimethylpolysiloxane, methylphenylpolysiloxane, methylhydrogenpolysiloxane and the like may be incorporated. However, since they have generally the anti-foaming effects, it is not preferable to incorporate them.

An amount of the oil to be incorporated is preferably 1-30% by mass, particularly 1-15% by mass in the whole cosmetic. When the amount is less than 1% by mass, a whipping base becomes hard, and it tends not to be easy to scoop by finger in some cases. When the amount exceeds 30% by mass, whipping ability is deteriorated and, moreover, extension on the skin is deteriorated in some cases.

Additionally, a nonionic surfactant as a foaming auxiliary agent may be incorporated. Examples of the nonionic surfactant include ether type surfactants such as polyoxyethylene 2-30 mole added {hereinafter, abbreviated as POE (2-30)}oleyl ether, POE(2-35)stearyl ether, POE(2-20)lauryl ether, POE(1-20)alkyl phenyl ether, POE(6-18)behenyl ether, POE(5-25)2-decyl pentadecyl ether, POE(3-20)2-decyl tetradecyl ether, POE(3-20)2-decyl tetradecyl ether, POE (8-16)2-octyl decyl ether and the like, ester type surfactants such as POE(4-60)hydrogenated castor oil, POE(3-14)fatty acid monoester, POE(6-30)fatty acid diester, POE(5-20)sorbitan fatty acid ester and the like, ethylene oxide added type surfactants such as ether ester type surfactants such as POE(2-30)glyceryl monoisostearate, POE(10-60)glyceryl triisostearate, POE(7-50)hydrogenated castor oil monoisostearate, POE(12-60)hydrogenated castor oil triisostearate and the like, polyglycerin fatty acid esters such as dacaglyceryl tetraoleate, hexaglyceryl triisostearate, tetraglyceryl diisostearate, diglyceryl diisostearate and the like, polyhydric alcohol fatty acid ester type surfactants such as glycerin fatty acid ester and the like such as glyceryl monoisostearate, glyceryl monooleate and the like, nonion-modified silicone surfactants, and the like.

Inter alias, 1 or 2 or more of polyglycerin (tri- or more-glycerin) fatty acid ester such as decaglyceryl tetraoleate, hexaglyceryl triisostearate, tetraglyceryl diisostearate and the like, POE added ether type surfactants such as POE(2-12) oleyl ether, POE(3-12)stearyl ether, POE(2-10)lauryl ether, POE(2-10)nonyl phenyl ether, POE(6-15)behenyl ether, POE(5-20)2-decyl pentadecyl ether, POE(5-17)2-decyl tetradecyl ether, POE(8-16)2-octyl decyl ether and the like, POE added ester type surfactants such as POE(10-20)hydrogenated castor oil, POE(5-14)oleic acid monoester, POE(6-20) oleic acid diester, POE(5-10)sorbitan oleic acid ester and the like, POE added ether ester type surfactants such as POE (3-15)glyceryl monoisostearate, POE(10-40)glyceryl triisostearate and the like, as well as ethylene oxide added type nonionic surfactants are suitably used. Alternatively, cationic surfactants and amphoteric surfactants may be incorporated in such a range that the effects of the present invention are not deteriorated.

B. Whipped O/W type emulsion cosmetic containing a nonionic surfactant

Examples of the nonionic surfactant used in the present invention include sucrose fatty acid ester, glycerin fatty acid ester, sorbitan fatty acid ester and POE hydrogenated castor oil. Embodiments of the nonionic surfactant include ethylene oxide added surfactants such as ether type surfactants such as polyoxyethylene 2-30 mole added {abbreviated as POE(2-30)}oleyl ether, POE(2-35)stearyl ether, POE(2-20)lauryl ether, POE(1-20)alkyl phenyl ether, POE(6-18)behenyl ether, POE(5-25)2-decyl pentadecyl ether, POE(3-20)2-decyl tetradecyl ether, POE(3-20)2-decyl tetradecyl ether, POE(8-16)2-octyl decyl ether and the like, ester type surfactants such as POE(4-60)hydrogenated castor oil, POE(3-14)fatty acid monoester, POE(6-30)fatty acid diester, POE(5-20)sorbitan fatty acid ester and the like, ether ester type surfactants such as POE(2-30)glyceryl monoisostearate, POE(10-60)glyceryl triisostearate, POE(7-50)hydrogenated castor oil monoisostearate, POE(12-60)hydrogenated castor oil triisostearate and the like, ester type surfactants such as polyhydric alcohol fatty acid esters such as polyglycerin fatty acid ester such as decaglyceryl tetraoleate, hexaglyceryl triisostearate, tetraglyceryl diisostearate, diglyceryl diisostearate and the like, polyhydric alcohol fatty acid ester such as glycerin fatty acid esters such as glyceryl monoisostearate, glyceryl monooleate and the like, sucrose mono-octastearic acid ester and the like. Alternatively, cationic surfactants and amphoteric surfactants may be incorporated in such a range that the effects of the present invention are not deteriorated. In the case where fine foams and high overrun degree are neceessary, it is desirable that casein and/or lecithin are contained. Examples of the lecithin include commercially available soybean lecithins, and their purified lecithins. Casein may be added at the powder form or the solution in water form. Casein may be a sodium salt or a potassium salt.

An amount of the nonionic surfactant, casein and lecithin to be added is preferably 0.1-15% by mass, in particular 0.5-10% by mass in the whole cosmetic. When the amount is less than 0.1% by mass, sufficient foaming cannot be obtained in some cases. When the amount exceeds 15% by mass, odor of a base is deteriorated at an elevated temperature and, moreover, the stickiness is felt, the cosmetic is not preferable in the safety for the skin in some cases.

The solid oil used in the present invention is an oil which is a solid at a temperature of 30°C or lower, preferably 50°C or lower, and which has IOB being not zero. Since non-polar oils having IOB being zero, such as liquid paraffin and vaseline deteriorate whipping ability, it is desirable that an amount thereof to be incorporated is as small as possible. By incorporation of an oil, a whipped O/W type emulsion containing a gelling agent does not become too hard and retains sufficient softness for scooping with finger. An amount of the oil to be incorporated is 1-30% by mass, particularly 1-15% by mass in the whole cosmetic. When the amount is less than 1% by mass, since a whipping base becomes hard, it tends not to be easy to scoop by finger in some cases. When the amounts exceed 30% by mass, whipping ability is deteriorated and, moreover, extension on the skin is deteriorated in some cases.

Example of the solid oil include solid fats and oils such as cacao fat, coconut oil, horseflesh tallow, hydrogenated coconut oil, palm oil, beef tallow, ram tallow, hardened beef tallow, palm kernel oil, lard tallow, beef bone tallow, Japan wax kernel oil, hardened oil, beef leg tallow, Japan wax, hydrogenated castor oil and the like, waxes such as beeswax, candelilla wax, cotton wax, carnauba wax, baybeny wax, insect wax, spermaceti, montan wax, bran wax, lanolin, kapok wax, lanolin acetate, sugarcane wax, lanolin fatty acid isopropyl, hexyl laurate, reduced lanolin, jojoba wax, hard lanolin, shellac wax, POE lanolin alcohol ether, POE lanolin alcohol acetate, lanolin fatty acid polyethylene glycol, POE hydrogenated lanolin alcohol ether and the like.

In addition to the aforementioned solid oils, liquid oils such as liquid lanolin, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid ester, N-alkyl glycol monoisostearate, neopentyl glycol dicaprate, diisostearyl malate, glycerin di-2-heptylundecanoate, trimethylolpropane tri-2-ethylhexylate, trimethylolpropane triisostearate, pentaerythritol tetra-2-ethylhexylate, glycerin tri-2-ethylhexylate, trimethylolpropane triisostearate, cetyl-2-ethyl hexanoate, 2-ethylhexyl palmitate, glycerin trimyristate, glyceride tri-2-heptylundecanoate, castor oil fatty acid methyl ester, oleic acid oil, cetostearyl alcohol, acetoglyceride, 2-heptylundecyl palmitate, diisopropyl adipate, 2-octyl dodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, triethyl citrate and the like, higher alcohols such as stearyl alcohol, behenyl alcohol, oleyl alcohol and the like, oil-soluble vitamins such as vitamin A and the like and derivatives thereof, sterols, natural and synthetic perfumes, ultraviolet absorbing agent, and water-slightly soluble substances may be incorporated in an oily phase.

If it is a small amount, hydrocarbon oils having IOB being zero, such as liquid paraffin, ozocerite, squalene, pristane. paraffin, ceresine, squalane, vaseline, microcrystalline wax and the like may be incorporated.

In addition to these oily ingredients, a small amount of silicone oil such as straight polysiloxane and the like such as dimethylpolysiloxane, methylphenylpolysiloxane, methylhydrogenpolysiloxane and the like can be incorporated. However, since they have the anti-foaming effects, it is not preferable to incorporate them.

An amount of solid oil to be incorporated is preferably 20% by mass or more, particularly 40% by mass or more in the whole oil in the cosmetic. When the amount is less than 20% by mass, the foam stability at a temperature of 30°C or higher is not retained in some cases.

The aforementioned 1 or 2 whipped O/W type emulsion cosmetic may further contain powders. By incorporation of powders, the crunchy feeling may be added to a whipped base. Examples of the powders include talc, titanium oxide, zinc oxide, polyethylene powders, nylon powders, starch powders and the like. If a small amount, silicone powders may be incorporated. However, since whipping ability is deteriorated, it is not preferable to incorporate them.

In addition, polyhydric alcohols and humectants may be incorporated into the present whipped O/W type emulsion cosmetic to enhance the moisture-retaining effects as far as the effects of the present invention are not deteriorated.

Examples of the polyhydric alcohol include divalent alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol and the like, trivalent alcohols, glycerin, trimethylolpropane, 1,2,6-hexanetriol and the like, tetravalent alcohols such as pentaerythritol and the like, pentavalent alcohols such as xylitol and the like, hexavalent alcohols such as sorbitol, mannitol and the like, multivalent alcohol copolymers such as diethylene glycol, dipropylene glycol, triethylene glycol, polypropylene glycol, triglycerin, tetraglycerin, polyglycerin and the like, divalent alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methyl hexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether and the like, divalent alcohol alkyl ethers such as diethylene glycol monomethyl ether, diethylene glycol monoethyl ether, diethylene glycol monobutyl ether, diethylene glycol, dimethyl ether, diethylene glycol diethyl ether, diethylene glycol dibutyl ether, diethylene glycol methyl ethyl ether, triethylene glycol monomethyl ether, triethylene glycol monoethyl ether, triethylene glycol monoethyl ether, propylene glycol monomethyl ether, propylene glycol monoethyl ether, propylene glycol monobutyl ether, propylene glycol monoisopropyl ether, dipropylene glycol methyl ether, dipropylene glycol ethyl ether, dipropylene glycol butyl ether and the like, divalent alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, ethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate and the like, glycerin monoalkyl ethers such as chimyl alcohol, selachyl alcohol, batyl alcohol and the like, sugar alcohols such as sorbitol, maltitol, maltotriose, mannitol, sucrose, erythritol, glucose, fructose, starch-degraded sugar, maltose, xylitol, starch-degraded sugar reduced alcohol and the like, glysolid, tetrahydrofurfuryl alcohol, POE tetrahydrofurfuryl alcohol, POP butyl ether, POP • POE butyl ether, polyoxypropylene glycerin ether, POP glycerin ether, POP glycerin ether phosphate, POP • POE pentaerythritol ether and the like.

Examples of the humectant include chondroitin sulfate, hyaluronic acid, mucoitin sulfate, charonic acid, atellocollagen, cholesteryl-12-hydroxy stearate, sodium lactate, bile acid mono-salt, d,l-pyrrolidonecarbooxylic acid mono-salt, short chain soluble collagen, Rosa roxburghii extract, yarrow extract and the like.

In addition, other water-soluble polymers may be incorporated in such a range that the use feeling, pH and the like of the present cosmetics are not deteriorated. Examples of the natural water-soluble polymer include plant series polymers such as gum arabic, tragacanth gum, galactan, guar gum, carob gum, caraya gum, pectin, quince seed, algae colloid (seaweed extract), starch (rice, corn, potato, wheat), glycyrrhizic acid and the like, microbial series polymers such as xanthan gum, dextran, succinoglucan, pullulan and the like, and animal series polymers such as collagen, casein, albumin and the like.

Examples of the semi-synthetic water-soluble polymers include starch series polymers such as carboxymethylstarch, methylhydroxystarch and the like, cellulose series polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose (CMC), crystalline cellulose, cellulose powder and the like, alginic acid series polymers such as propylene glycol alginate and the like.

Examples of the synthetic water-soluble polymers include vinyl series polymers such as polyvinyl alcohol, polyvinyl methyl ether series polymer, polyvinyl pyrrolidone, carboxyvinyl polymer (CARBOPOL 940, 941; BF Goodrich) and the like, polyoxyethylene series polymers such as polyethylene glycol 20,000, polyethylene glycol 6,000, polyethylene glycol 4,000 and the like, copolymer series polymers such as polyoxyethylene polyoxypropylene copolymer and the like, acrylic series polymers such as sodium polyacrylate, polyethylacrylate, polyacrylamide and the like, polyethyleneimine and the like.

As for the present cosmetic, various ingredients which are usually incorporated in the field of cosmetics and pharmaceuticals may be incorporated. Among such ingredients, examples of the ingredients in aqueous phase include water-soluble active substances such as vitamins such as vitamin B group, vitamin C and derivatives thereof, pantothenic acid and derivatives thereof, biotin and the like, buffers such as sodium glutamate, arginine, aspartic acid, citric acid, tartaric acid and lactic acid, various plant extracts, chelating agents such as EDTA as well as water-soluble ultraviolet absorbing agents, various pigments and the like.

The present O/W type emulsion cosmetic is utilized, for example, as a skin cosmetic, a hair cosmetic and a skin external preparation in the cosmetic quasi-drug or medicinal field. Since the present cosmetic has the excellent usability, it is desirably used as an emulsion cosmetic.

Examples of the device for making a base contain bubbles are not particularly limited so long as the device takes bubbles therein, but include a bench batch mixer for cooking, dasher and the like.

The present whipped O/W type emulsion cosmetic may be prepared by whipping an O/W type emulsion containing a polymer which forms a gel having no fluidity, or by whipping an O/W type emulsion containing no polymer which forms a gel having no fluidity and, thereafter, incorporating a polymer which forms a gel having no fluidity.

Then, a process of developing the present invention will be explained in more detail by way of Test Examples. All incorporation amounts mean percentage by mass. An incorporation amount in an aqueous phase and an incorporation amount are indicated, they are described expressly each every time. And, in other cases, an incorporation amount relative to the whole cosmetic is meant.

Methods of calculating overrun degree and assessment criteria therefor are described below prior to Test Examples. Overrun degree was calculated according to the following equation and assessed by the following assessment criteria:

$$\text{Overrun degree} = \{(A-B)/B\} \times 100$$

A: Cream weight at constant volume
B: Whipped cream weight at constant volume

<Overrun degree>

◎ Overrun degree is 100% or more.
○ Overrun degree is 50 or more to less than 100% .
△ Overrun degree is 10 or more to less than 50%.
× Overrun degree is less than 10%.

<Assessment of foam stability>

The foam stabilities after retained at 0 ˚C and 37˚C for 1 month were assessed with eyes according to the following criteria:

(Particle diameter of foam)

◎ Change is not observed at all.
○ Change diameter is hardly observed.
△ Increase in a particle diameter is observed.
× Increase in a particle diameter is remarkably observed.

(Disappearance of foam)

◎ Change is not observed at all.
○ Change is hardly observed.
△ Disappearance of foams is observed.
× Disappearance of foams is remarkably observed.

<Assessment of usability >

Ten Japanese female panelists of 20-35 years old took each whipped O/W type emulsion cosmetic immediately after preparation with a finger and organoleptically assessed the feeling of scoopability with a finger and the extensibility on the skin according to the following criteria:

(Scoopability with a finger)

◎ 8 or more panelists answered that scooping is easy.

○ 6 or 7 panelists answered that scooping is easy.
Δ 4 or 5 panelists answered that scooping is easy.
× 3 or fewer panelists answered that scooping is easy.

(Extensibility on the skin)

◎ 8 or more panelists answered that extension is better.
○ 6 or 7 panelists answered that extension is better.
Δ 4 or 5 panelists answered that extension is better.
× 3 or fewer panelists answered that extension is better.

A. Whipped O/W type emulsion cosmetic containing a higher fatty acid soap

Whipped O/W type emulsion cosmetics having the formulations in Table 1 was prepared, and overrun degree and the foam stability were studied.

Table 1

| Ingredient | Test Example | | | | | | |
|---|---|---|---|---|---|---|---|
| | 1 | 2 | 3 | 4 | 5 | 6 | 7 |
| (1) Agar | - | 2.0 | - | 2.0 | - | - | 2.0 |
| (2) Glucomannan | - | - | 1.0 | - | - | - | - |
| (3) Xanthan gum | - | - | 1.0 | - | 2.0 | - | - |
| (4) Carboxymethylcellulose | - | - | - | - | - | 2.0 | - |
| (5) Cationated hydroxycellulse | - | - | 0.5 | 0.5 | - | - | - |
| (6) 1,3-butyleneglycol | 5.0 | 5.0 | 5.0 | 5.0 | 5.0 | 5.0 | 5.0 |
| (7) Methylparaben | 0.2 | 0.2 | 0.2 | 0.2 | 0.2 | 2.0 | 2.0 |
| (8) Purified water | Balance | | | | | | |
| (9) Potassium hydroxide | 0.7 | 0.7 | 0.7 | 0.7 | 0.7 | 0.7 | - |
| (10) Sodium hexametaphosphate | 0.05 | 0.05 | 0.05 | 0.05 | 0.05 | 0.05 | 0.05 |
| (11) Stearic acid | 5.0 | 5.0 | 5.0 | 5.0 | 5.0 | 5.0 | - |
| (12) Palmitic acid | 5.0 | 5.0 | 5.0 | 5.0 | 5.0 | 5.0 | - |
| (13) POE hydrogenated castor oil | - | - | - | - | - | - | 10.0 |
| (14) 2-octyl dodecanol | 8.0 | 8.0 | 8.0 | 8.0 | 8.0 | 8.0 | 8.0 |
| <Overrun degree> | ◎ | ◎ | ◎ | ◎ | ◎ | ◎ | Δ |
| < Foam stability> | | | | | | | |
| 0˚C   Particle diameter of foam | ◎ | ◎ | ◎ | ◎ | ◎ | ◎ | ◎ |
| Disappearance of foam | ◎ | ◎ | ◎ | ◎ | ◎ | ◎ | ◎ |
| 37˚C   Particle diameter of foam | × | Δ | ◎ | ◎ | × | × | Δ |
| Disappearance of foam | × | Δ | ◎ | ◎ | × | × | Δ |

(Preparation)

Ingredients (1)-(8), and (10) were mixed and dissolved, and uniformly dispersed at 80˚C. To the aqueous phase were added (11)-(14) which had been uniformly dissolved at 80˚C and (9) which had been dissolved in a part of (8), which was uniformly dispersed with a homomixer, whipped with a batch mixer to prepare a whipped O/W type emulsion cosmetic, which was filled in a container at about 50˚C and cooled.

(Results)

Test Example 1 is a composition containing no gelling agent, and Test Examples 2-7 are a composition containing a polymer of a gelling agent. From the results in Table 1, it can be seen that the whipped O/W type emulsion cosmetics of Test Example 1 containing no polymer gelling agent is inferior in the foam stability at 37˚C. To the contrary, improvement in the foam stability at 37˚C was observed in whipped O/W type emulsion cosmetics of Test Examples 2-4 containing a polymer (agar, glucomannan) for foaming a gel having no fluidity. Here, glucomannan does not foam a gel only by itself, but by using together with xanthan gum, it forms a gel having no fluidity. In addition, it can be seen that Test Example 4 containing, in particular, a cationic polymer is the most excellent in the foam stability at 37˚C. However, in Test Examples 5 and 6 in which only xanthan gum or carboxymethylcellulose which has the viscosity-increasing effects but does not form a gel having no fluidity is incorporated, the foam stability at 37˚C was inferior. Therefore, it can be seen that, by incorporation of a polymer which forms a gel having no fluidity, the foam stability at an elevated temperature is improved. In addition, Test Example 7 in which a nonionic surfactant is incorporated in place of a higher fatty acid soap has low overrun degree and is also inferior in the foam stability at 37˚C.

A-1 Amount of a polymer which forms a gel having no fluidity to be incorporated

Whipped O/W type emulsion cosmetics having the formulations in Table 2 were prepared, and an amount of a polymer which forms a gel having no fluidity, to be incorporated in the present invention was studied.

Table 2

| Ingredient | Test Example | | | | | | |
|---|---|---|---|---|---|---|---|
| | 8 | 9 | 10 | 11 | 12 | 13 | 14 |
| (Aqueous phase) | | | | | | | |
| Agar | 0.01 | 0.1 | 0.8 | 2.0 | 5.0 | 10.0 | 15.0 |
| Cationated hydroxycellulose | 0.5 | 0.5 | 0.5 | 0.5 | 0.5 | 0.5 | 0.5 |
| 1,3-butyleneglycol | 5.0 | 5.0 | 5.0 | 5.0 | 5.0 | 5.0 | 5.0 |
| Methylparaben | 0.2 | 0.2 | 0.2 | 0.2 | 0.2 | 2.0 | 2.0 |
| Purified water | Balance | | | | | | |
| Sodium hexametaphosphate | 0.05 | 0.05 | 0.05 | 0.05 | 0.05 | 0.05 | 0.05 |
| (Oil phase) | | | | | | | |
| Stearic acid | 5.0 | 5.0 | 5.0 | 5.0 | 5.0 | 5.0 | 5.0 |
| Palmitic acid | 5.0 | 5.0 | 5.0 | 5.0 | 5.0 | 5.0 | 5.0 |
| Potassium hydroxide | 0.7 | 0.7 | 0.7 | 0.7 | 0.7 | 0.7 | 0.7 |
| 2-octyldodecanol | 8.0 | 8.0 | 8.0 | 8.0 | 8.0 | 8.0 | 8.0 |
| Amount of a polymer which foams a gel having no fluidity to be incorporated_in aqueous phase | | | | | | | |
| | 0.01 | 0.12 | 0.98 | 2.46 | 6.15 | 12.3 | 18.5 |
| < Overrun degree > | ◎ | ◎ | ◎ | ◎ | ◎ | ○ | ○ |
| < Foam stability > | | | | | | | |
| 37˚C    Particle diameter of foam | Δ | ○ | ◎ | ◎ | ◎ | ◎ | ◎ |
| Disappearance of foam | Δ | ○ | ◎ | ◎ | ◎ | ◎ | ◎ |
| <Usability> | | | | | | | |
| Scoopability with a finger | ◎ | ◎ | ◎ | ◎ | ◎ | ○ | Δ |
| Extensibility on the skin | ○ | ◎ | ◎ | ◎ | ◎ | ○ | ○ |

(Results)

From the results in Table 2, it can be seen that, when an amount of a polymer which forms a gel having no fluidity to be incorporated is less than about 0.1% by mass in an aqueous phase, the foam stability at 37˚C is inferior. In addition, it can be seen that, when an amount of a polymer which forms a gel having no fluidity to be incorporated exceeds 13% by mass, the foam stability at 37˚C is inferior and scoopability with a finger is also inferior. Therefore, it can be seen that an amount of a polymer which forms a gel having no fluidity to be incorporated is suitably about 0.1-13% by mass, more suitably about 1-7% by mass in an aqueous phase.

A-2 Amount of a higher fatty acid soap to be incorporated

Whipped O/W type emulsion cosmetics having the formulations in Table 3 was prepared, and an amount of a higher fatty acid to be incorporated was studied.

Table 3

| Ingredient | | Test Example | | | | | | |
|---|---|---|---|---|---|---|---|---|
| | | 15 | 16 | 17 | 18 | 19 | 20 | 21 |
| Agar | | 2.0 | 2.0 | 2.0 | 2.0 | 2.0 | 2.0 | 2.0 |
| Cationated hydroxycellulose | | 0.5 | 0.5 | 0.5 | 0.5 | 0.5 | 0.5 | 0.5 |
| 1,3-butyleneglycol | | 5.0 | 5.0 | 5.0 | 5.0 | 5.0 | 5.0 | 5.0 |
| Methylparaben | | 0.2 | 0.2 | 0.2 | 0.2 | 0.2 | 2.0 | 2.0 |
| Purified water | | Balance | | | | | | |
| Sodium hexametaphosphate | | 0.05 | 0.05 | 0.05 | 0.05 | 0.05 | 0.05 | 0.05 |
| Stearic acid | | 0.5 | 1.0 | 5.0 | 10.0 | 15.0 | 20.0 | 25.0 |
| Potassium hydroxide | | 0.035 | 0.07 | 0.35 | 0.7 | 1.05 | 1.4 | 1.75 |
| 2-octyldodecanol | | 8.0 | 8.0 | 8.0 | 8.0 | 8.0 | 8.0 | 8.0 |
| <Overrun degree> | | Δ | ○ | ◎ | ◎ | ◎ | ◎ | ◎ |
| < Foam stability > | | | | | | | | |
| 37˚C | Particle diameter of foam | Δ | ○ | ◎ | ◎ | ◎ | ◎ | ◎ |
| | Disappearance of foam | Δ | ○ | ◎ | ◎ | ◎ | ◎ | ◎ |
| < Usability > | | | | | | | | |
| | Scoopability with a finger | Δ | ○ | ◎ | ◎ | ◎ | ○ | ○ |
| | Extensibility on the skin | Δ | ○ | ◎ | ◎ | ◎ | ○ | Δ |

(Results)

From the results in Table 3, it can be seen that, when an amount of a higher fatty acid soap to be incorporated is less than 1% by mass, overrun degree, the foam stability at 37˚C and the usability are inferior. In addition, it can be seen that, when the amount is greater than 20% by mass, the usability is inferior. Therefore, it can be seen that an amount of a higher fatty acid soap is suitably about 1-20% by mass, more suitably about 5-15% by mass.

A-3 Amount of a cationic polymer to be incorporated

By using whipped O/W type emulsion cosmetics having the formulations in Table 4, an amount of a cationic polymer to be incorporated in the present invention was studied.

Table 4

| Ingredient | | Test Example | | | | | | |
|---|---|---|---|---|---|---|---|---|
| | | 22 | 23 | 24 | 25 | 26 | 27 | 28 |
| Agar | | 2.0 | 2.0 | 2.0 | 2.0 | 2.0 | 2.0 | 2.0 |
| Cationated hyclioxycellulose | | 0.005 | 0.01 | 0.05 | 1.0 | 3.0 | 5.0 | 7.0 |
| 1,3-butyleneglycol | | 5.0 | 5.0 | 5.0 | 5.0 | 5.0 | 5.0 | 5.0 |
| Methylparaben | | 0.2 | 0.2 | 0.2 | 0.2 | 0.2 | 2.0 | 2.0 |
| Purified water | | Balance | | | | | | |
| Sodium hexametaphosphate | | 0.05 | 0.05 | 0.05 | 0.05 | 0.05 | 0.05 | 0.05 |
| Stearic acid | | 5.0 | 5.0 | 5.0 | 5.0 | 5.0 | 5.0 | 5.0 |
| Palmitic acid | | 5.0 | 5.0 | 5.0 | 5.0 | 5.0 | 5.0 | 5.0 |
| Potassium hydroxide | | 0.7 | 0.7 | 0.7 | 0.7 | 0.7 | 0.7 | 0.7 |
| 2-octyldodecanol | | 8.0 | 8.0 | 8.0 | 8.0 | 8.0 | 8.0 | 8.0 |
| < Overrun degree > | | ◎ | ◎ | ◎ | ◎ | ◎ | ◎ | ◎ |
| < Foam stability > | | | | | | | | |
| 37˚C | Particle diameter of foam | Δ | ○ | ◎ | ◎ | ◎ | ◎ | ◎ |
| | Disappearance of foam | Δ | ○ | ◎ | ◎ | ◎ | ◎ | ◎ |
| < Usability > | | | | | | | | |

(continued)

| Ingredient | Test Example | | | | | | |
|---|---|---|---|---|---|---|---|
| | 22 | 23 | 24 | 25 | 26 | 27 | 28 |
| Scoopability with a finger | ◎ | ◎ | ◎ | ◎ | ◎ | ○ | Δ |
| Extensibility on the skin | ◎ | ◎ | ◎ | ◎ | ◎ | ○ | Δ |

(Results)

From the results in Table 4, it can be seen that, when an amount of a cationic polymer to be incorporated is less than 0.01% by mass, the foam stability at 37˚C is inferior. In addition, it can be seen that, when the amount is greater than 5% by mass, the usability is inferior. Therefore, it can be seen that an amount of a cationic polymer to be incorporated is suitably about 0.01-5% by mass, more suitably 0.05-3% by mass.

A-4 Amount of an oil to be incorporated

Whipped O/W type emulsion cosmetics having the formulations in Table 5 was prepared, and an amount of an oil to be incorporated was studied.

Table 5

| Ingredient | | Test Example | | | | | |
|---|---|---|---|---|---|---|---|
| | | 29 | 30 | 31 | 32 | 33 | 34 |
| Agar | | 2.0 | 2.0 | 2.0 | 2.0 | 2.0 | 2.0 |
| Cationated hydroxycellulose | | 0.5 | 0.5 | 0.5 | 0.5 | 0.5 | 0.5 |
| 1,3-butyleneglycol | | 5.0 | 5.0 | 5.0 | 5.0 | 5.0 | 5.0 |
| Methylparaben | | 0.2 | 0.2 | 0.2 | 0.2 | 0.2 | 0.2 |
| Purified water | | Balance | | | | | |
| Sodium hexametaphosphate | | 0.05 | 0.05 | 0.05 | 0.05 | 0.05 | 0.05 |
| Behenic acid | | 5.0 | 5.0 | 5.0 | 5.0 | 5.0 | 5.0 |
| Palmitic acid | | 5.0 | 5.0 | 5.0 | 5.0 | 5.0 | 5.0 |
| Potassium hydroxide | | 0.7 | 0.7 | 0.7 | 0.7 | 0.7 | 0.7 |
| Isostearyl alcohol | | 0.1 | 1.0 | 8.0 | 15.0 | 30.0 | 35.0 |
| < Overrun degree > | | ◎ | ◎ | ◎ | ◎ | ○ | Δ |
| < Foam stability > | | | | | | | |
| 37˚C | Particle diameter of foam | ○ | ◎ | ◎ | ◎ | ◎ | ◎ |
| | Disappearance of foam | ○ | ◎ | ◎ | ◎ | ◎ | ◎ |
| < Usability > | | | | | | | |
| | Scoopability with a finger | ○ | ◎ | ◎ | ◎ | ◎ | ○ |
| | Extensibility on the skin | × | ○ | ◎ | ◎ | ○ | Δ |

(Results)

From the results in Table 5, it can be seen that, when an amount of an oil to be incorporated is less than 1% by mass, the usability is inferior. It can be seen that, when the amount is greater than 30% by mass, since whipping becomes worse, overrun degree is decreased and the usability is inferior. Therefore, it can be seen that an amount of an oil to be incorporated is suitably 1-30% by mass, more suitably 8-15% by mass.

Table 6

| Ingredient | Test Example | | | | | | | |
|---|---|---|---|---|---|---|---|---|
| | 35 | 36 | 37 | 38 | 39 | 40 | 41 | 42 |
| Stearic acid | 5.0 | 5.0 | - | 5.0 | 1.0 | 5.0 | 5.0 | - |
| Palmitic acid | 5.0 | - | 6.0 | - | - | - | 5.0 | 5.0 |
| Behenic acid | - | 5.0 | 6.0 | 5.0 | - | 5.0 | - | 5.0 |

(continued)

| Ingredient | Test Example | | | | | | | |
|---|---|---|---|---|---|---|---|---|
| | 35 | 36 | 37 | 38 | 39 | 40 | 41 | 42 |
| 2-octyldodecanol | 8.0 | 2.0 | 8.0 | - | 4.0 | 4.0 | 0.1 | 8.0 |
| Jojoba oil | - | 5.0 | - | - | - | - | - | - |
| Cyclic Silicone | - | - | - | - | - | 4.0 | - | - |
| POE hydrogenated castor oil | - | 0.3 | - | - | 2.0 | - | - | - |
| Agar | 2.0 | 2.0 | 1.0 | - | 2.0 | 2.0 | 2.0 | 2.0 |
| Gelatin | - | - | 1.0 | - | - | - | - | - |
| Xanthane gum | - | 0.1 | - | 3.0 | - | - | - | - |
| Cationated hydroxycellulose | 0.5 | 0.5 | 0.5 | 0.5 | 0.5 | 0.5 | 0.5 | 0.01 |
| 1,3-butyleneglycol | 5.0 | 5.0 | 5.0 | 5.0 | 5.0 | 5.0 | 5.0 | 5.0 |
| Methylparaben | 0.2 | 0.2 | 0.2 | 0.2 | 0.2 | 0.2 | 0.2 | 0.2 |
| Purified water | Balance | | | | | | | |
| Potassium hydroxide | 0.7 | 0.7 | 0.8 | 0.7 | 0.07 | 0.7 | 0.7 | 0.7 |
| Sodium hexametaphosphate | 0.05 | 0.05 | 0.05 | 0.05 | 0.05 | 0.05 | 0.05 | 0.05 |
| Talc | 2.0 | - | - | - | - | - | - | - |
| <Overrun degree (%) > | 150 | 160 | 150 | 150 | 20 | 20 | 190 | 150 |
| < Foam stability > | | | | | | | | |
| 0°C    Particle diameter of foam | ◎ | ◎ | ◎ | ◎ | ◎ | ◎ | ◎ | ◎ |
| Disappearance of foam | ◎ | ◎ | ◎ | ◎ | ◎ | ◎ | ◎ | ◎ |
| 37°C    Particle diameter of foam | ◎ | ◎ | ◎ | × | Δ | ◎ | Δ | Δ |
| Disappearance of foam | ◎ | ◎ | ◎ | × | Δ | ◎ | ◎ | Δ |
| <Usability> | | | | | | | | |
| Scoopability with a finger | ◎ | ◎ | ◎ | ◎ | Δ | × | ○ | ◎ |
| Extensibility on the skin | ◎ | ◎ | ◎ | ◎ | Δ | × | Δ | ◎ |

(Results)

From the results in Table 6, it can be seen that the present whipped O/W type emulsion cosmetic has the better foam stability and the excellent usability. In addition, from Test Example 40, it can be seen that, when a silicone oil is incorporated too much, overrun degree is decreased due to the anti-foaming effects, and the usability is inferior.

Example 1 Massage cleansing cosmetics

|  |  |
|---|---|
| (1) Purified water | Balance |
| (2) Methylparaben | 0.1 |
| (3) 1,3-butyleneglycol | 5.0 |
| (4) Ascorbic acid | 0.5 |
| (5) Cationated hydroxycellulose | 0.1 |
| (6) Xanthane gum | 1.0 |
| (7) Glucomannan | 1.0 |
| (8) Hydrophobing starch | 2.0 |
| (9) Potassium hydroxide | 0.8 |
| (10) Stearic acid | 3.0 |
| (11) Palmitic acid | 4.0 |
| (12) Behenic acid | 3.0 |
| (13) Cetyl octanoate | 5.0 |
| (14) Isostearyl alcohol | 4.0 |
| (15) Perfume | q.s. |

(Process for preparation)

Ingredients (1)-(7) were mixed and dissolved at 80˚C. To this was added (8), which was dispersed uniformly. To this aqueous phase were added uniformly dissolved (10)-(14) and (9) dissolved in a part of (1) at 80˚C, (15) was further added, which was uniformly dispersed with a homomixer, whipped with a batch mixer to prepare a whipped O/W type emulsion cosmetic, which was filled in a container at about 50˚C and cooled as it was. The resulting whipped cream had the better foam stability and the excellent usability.

Example 2 Moisture-retaining cream

| | |
|---|---|
| (1) Purified water | Balance |
| (2) Methylparaben | 0.1 |
| (3) Glycerin | 5.0 |
| (4) Trymethylglycine | 1.0 |
| (5) Cationated hydroxycellulose | 0.5 |
| (6) Hydoroxyethylcellulose | 0.5 |
| (7) Sucrose stearate | 2.0 |
| (8) Carrageenan | 2.0 |
| (9) Potassium hydroxide | 0.3 |
| (10) Stearic acid | 2.0 |
| (11) Palmitic acid | 2.0 |
| (12) 2-octyldodecanol | 4.0 |
| (13) Pentaerythrytyl tetraoctanoate | 4.0 |
| (14) Liquid paraffin | 2.0 |
| (15) Titanium oxide | 4.0 |
| (16) Perfume | q.s. |

(Process for preparation)

Ingredients (1)-(7) (aqueous phase) were mixed and dissolved at 80˚C. To this was added (15), which was uniformly dispersed. To this aqueous phase were added uniformly dissolved (10)-(14) (oily phase) and (9) dissolved in a part of (1) at 80˚C. (15) and (16) were further added thereto, which was uniformly dispersed with a homomixer, and whipped with a batch mixer to prepare a whipped O/W type emulsion cosmetic. (8) dissolved in a part of (1) was mixed in the whipped cream at 80˚C, which was filled in a container at about 50˚C and cooled as it was. The resulting whipped cream had the better foam stability and the excellent usability.

Example 3 Massage cleansing cosmetic

| | |
|---|---|
| (1) Purified water | Balance |
| (2) Phenoxyethanol | 0.1 |
| (3) Dipropyleneglycol | 5.0 |
| (4) Arbtin | 0.5 |
| (5) Sucrose stearate | 0.5 |
| (6) Polyoctanium | 0.1 |
| (7) Agar | 1.0 |
| (8) Nylon powder | 2.0 |
| (9) Potassium hydroxide | 0.8 |
| (10) Stearic acid | 3.0 |
| (11) Isostearic acid | 4.0 |
| (12) Behenic acid | 5.0 |

(continued)

| | |
|---|---|
| (13) Vaseline | 1.0 |
| (14) Isostearyl alcohol | 4.0 |
| (15) Perfume | q.s. |

(Process for preparation)

Ingredients (1)-(7) were mixed and dissolved at 80˚C. To this was added (8), which was uniformly dispersed. To this aqueous phase were added uniformly dissolved (10)-(14) and (9) dissolved in a part of (1) at 80˚C. (15) was further added, which was uniformly dispersed with a homomixer and whipped with a batch mixer to prepare a whipped O/W type emulsion cosmetic, which was filled in a container at about 50˚C and cooled as it was. The resulting whipped cream had the better foam stability and the excellent usability.

Example 4 Massage cream

| | |
|---|---|
| (1) Purified water | Balance |
| (2) Methylparaben | 0.1 |
| (3) 1,3-butyleneglycol | 5.0 |
| (4) Ascorbic acid | 0.5 |
| (5) Poly dimethylmethylenepiperidinium chloride | 0.5 |
| (6) Glucomannan | 1.0 |
| (7) Kertrol | 1.0 |
| (8) Sodium alginate | 1.0 |
| (9) Titanium oxide | 2.0 |
| (10) Potassium hydroxide | 0.8 |
| (11) Stearic acid | 3.0 |
| (12) Behenic acid | 4.0 |
| (13) Stearyl alcohol | 1.0 |
| (14) Cetyl octanoate | 5.0 |
| (15) Menthol | 0.05 |
| (16) Perfume | q.s. |

(Process for preparation)

Ingredients (1)-(8) were mixed and dissolved at 80˚C. To this was added (9), which was uniformly dispersed. To this aqueous phase were added uniformly dissolved (11)-(15) and (10) dissolved in a part of (1). (16) was further added thereto, which was uniformly dispersed with a homomixer, whipped with a batch mixer to prepare a whipped O/W type emulsion cosmetic, which was filled in a container at about 50˚C and cooled as it was. The resulting whipped cream had the better foam stability and the excellent usability.

Example 5 Daytime beauty lotion

| | |
|---|---|
| (1) Purified water | Balance |
| (2) Methylparaben | 0.1 |
| (3) Glycerin | 5.0 |
| (4) Hyaluronic acid | 0.5 |
| (5) Polyacrylate | 0.5 |
| (6) Gelatin | 1.0 |
| (7) Curdlan | 1.0 |
| (8) Zinc oxide | 2.0 |

(continued)

| | |
|---|---|
| (9) Potassium hydroxide | 0.8 |
| (10) Stearic acid | 3.0 |
| (11) Myristic acid | 1.0 |
| (12) Behenic acid | 2.0 |
| (13) Palm oil | 5.0 |
| (14) Ultraviolet absorbing agent | 1.0 |
| (15) Perfume | q.s. |

(Process for preparation)

Ingredients (1)-(7) were mixed and dissolved at 80˚C. To this was added (8), which was uniformly dispersed. To this aqueous phase were added uniformly dissolved (10)-(14) and (9) dissolved in a part of (1) at 80˚C. (15) was further added thereto, which was uniformly dispersed with a homomixer and whipped with a batch mixer to prepare a whipped O/W type emulsion cosmetic, which was filled in a container at about 50˚C and cooled as it was. The resulting whipped cream had the better foam stability and the usability.

B. Whipped O/W type emulsion cosmetic containing nonionic surfactant

Whipped O/W type emulsion cosmetics having the formulations in Tables 7 and 8 were prepared, and overrun degree, the foam stability and the usability were studied. Test Example 43 is a composition containing no gelling agent, and Test Examples 44-51 are a composition containing a polymer substance of a gelling agent.

Table 7

| Ingredient | | Test Example | | | |
|---|---|---|---|---|---|
| | | 43 | 44 | 45 | 46 |
| (1) Sucrose monostearate | | 1.0 | 1.0 | 1.0 | 1.0 |
| (2) Casein | | 1.0 | 1.0 | 1.0 | 1.0 |
| (3) Agar | | - | 2.0 | - | - |
| (4) Xanthane gum | | - | - | 2.0 | - |
| (5) Carboxymethylcellulose | | - | - | - | 2.0 |
| (6) 1,3-butyleneglycol | | 5.0 | 5.0 | 5.0 | 5.0 |
| (7) Methylparaben | | 0.2 | 0.2 | 0.2 | 0.2 |
| (8) Purified water | | | Balance | | |
| (9) Sodium hexametaphosphate | | 0.05 | 0.05 | 0.05 | 0.05 |
| (10) Palm hydrogenated oil | | 10.0 | 10.0 | 10.0 | 10.0 |
| (11) Beeswax | | 10.0 | 10.0 | 10.0 | 10.0 |
| (12) Jojoba oil | | - | - | - | - |
| (13) Vaseline | | - | - | - | - |
| (14) Soybean lecithin | | 0.1 | 0.1 | 0.1 | 0.1 |
| < Overrun degree> | | ◎ | ◎ | ◎ | ◎ |
| < Foam stability > | | | | | |
| 37˚C | Particle diameter of foam | × | ◎ | × | × |
| | Disappearance of foam | × | ◎ | × | × |
| < Usability > | | | | | |
| | Scoop ability with a finger | ◎ | ◎ | ◎ | ◎ |
| | Extensibility on the skin | ◎ | ◎ | ◎ | ◎ |

(Process for preparation)

To an aqueous phase in which ingredients (1)-(9) were mixed and dissolved at 80˚C, were added (10)-(14) dissolved uniformly at 80˚C, which was uniformly dispersed with a homomixer, and whipped with a batch mixer at 40˚C to prepare a whipped O/W type emulsion cosmetic, which was filled in a container at about 50˚C and cooled as it was. The following Test Examples were performed according to the same manner as that described above.

(Results)

From the results in Table 7, it can be seen that Test Example 43 containing no polymer which forms a gel having no fluidity and a melting point of 40˚C or higher which forms a gel, is inferior in the foam stability. To the contrary, it can be seen that Test Example 44 containing a polymer which forms a gel having no fluidity and a melting point of 40˚C or higher, and solid oils, and a nonionic surfactant is excellent in all respects including in the foam stability. However, it can be seen that Test Examples 45 and 46 containing a polymer which forms a gel having the fluidity are inferior in the foam stability.

Table 8

| Ingredient | Test Example | | | | |
|---|---|---|---|---|---|
| | 47 | 48 | 49 | 50 | 51 |
| (1) Sucrose monostearate | 1.0 | 1.0 | 1.0 | 2.1 | 2.1 |
| (2) Casein | 1.0 | 1.0 | 1.0 | 1.0 | - |
| (3) Glucomannan | 1.0 | 1.0 | 1.0 | 1.0 | 1.0 |
| (4) Xanthane gum | 1.0 | 1.0 | 1.0 | 1.0 | 1.0 |
| (5) Carboxymethylcellulose | - | - | - | - | - |
| (6) 1,3-butyleneglycol | 5.0 | 5.0 | 5.0 | 5.0 | 5.0 |
| (7) Methylparaben | 0.2 | 0.2 | 0.2 | 0.2 | 0.2 |
| (8) Purified water | Balance | | | | |
| (9) Sodium hexametaphosphate | 0.05 | 0.05 | 0.05 | 0.05 | 0.05 |
| (10) Palm hydrogenated oil | - | - | - | 10.0 | 10.0 |
| (11) Beeswax | - | - | - | 10.0 | 10.0 |
| (12) Jojoba oil | - | 20.0 | - | - | - |
| (13) Vaseline | - | - | 20.0 | - | - |
| (14) Soybean lecithin | 0.1 | 0.1 | 0.1 | - | 0.1 |
| < Overrun degree > | ◎ | ○ | Δ | ○ | ○ |
| < Foam stability > | | | | | |
| 37˚C  Particle diameter of foam | ◎ | × | × | ◎ | ◎ |
| Disappearance of foam | ◎ | Δ | Δ | ○ | ○ |
| <Usability> | | | | | |
| Scoop ability with a finger | × | ◎ | ◎ | ◎ | ◎ |
| Extensibility on the skin | × | ◎ | ◎ | ◎ | ◎ |

(Results)

Although an aqueous solution containing only glucomannan does not form a gel, by the use with xanthan gum, it forms a gel having no fluidity.

From the results in Table 8, Test Example 47 containing no oil is inferior in the usability. In addition, Test Example 48 containing a liquid oil in place of a solid oil, Test Example 49 containing a solid oil having IOB being zero are inferior in the foam stability. Futhermore, in Test Examples 50 and 51 containing neither lecithin nor casein, overrun degree was slightly low and the foam stability was slightly inferior. Therefore, it is preferable that lecithin and casein are incorporated in a nonionic surfactant.

B-1 Amount of a polymer which forms a gel having no fluidity and its melting point of 40˚C or higher to be incorporated

Whipped O/W type emulsion cosmetics having the formulations in Table 9 were prepared and an amount of a polymer to be incorporated was studied. The results of assessment are also shown.

Table 9

| Ingredient | Test Example | | | | | | |
|---|---|---|---|---|---|---|---|
| | 52 | 53 | 54 | 55 | 56 | 57 | 58 |
| (Aqueous phase) | | | | | | | |
| Sucrose monostearate | 1.0 | 1.0 | 1.0 | 1.0 | 1.0 | 1.0 | 1.0 |
| Casein | 1.0 | 1.0 | 1.0 | 1.0 | 1.0 | 1.0 | 1.0 |
| Agar | 0.01 | 0.1 | 0.8 | 2.0 | 5.0 | 10.0 | 15.0 |
| 1,3-butyleneglycol | 5.0 | 5.0 | 5.0 | 5.0 | 5.0 | 5.0 | 5.0 |
| Methylparaben | 0.2 | 0.2 | 0.2 | 0.2 | 0.2 | 0.2 | 0.2 |
| Purified water | | | | Balance | | | |
| Sodium hexametaphosphate | 0.05 | 0.05 | 0.05 | 0.05 | 0.05 | 0.05 | 0.05 |
| (Oil phase) | | | | | | | |
| Palm hydrogenated oil | 10.0 | 10.0 | 10.0 | 10.0 | 10.0 | 10.0 | 10.0 |
| Beeswax | 10.0 | 10.0 | 10.0 | 10.0 | 10.0 | 10.0 | 10.0 |
| Soybean lecithin | 0.1 | 0.1 | 0.1 | 0.1 | 0.1 | 0.1 | 0.1 |
| Amount of a polymer which forms a gel having no fluidity and its melting point of 40˚C or higher, to be incorporated in aqueous phase | | | | | | | |
| | 0.01 | 0.13 | 1.00 | 2.50 | 6.26 | 12.5 | 18.8 |
| <Overrun degree> | ◎ | ◎ | ◎ | ◎ | ◎ | ○ | ○ |
| < Foam stability> | | | | | | | |
| 37˚C Particle diameter of foam | Δ | ○ | ◎ | ◎ | ◎ | ◎ | ◎ |
| Disappearance of foam | Δ | ○ | ◎ | ◎ | ◎ | ◎ | ◎ |
| < Usability > | | | | | | | |
| Scoop ability with a finger | ◎ | ◎ | ◎ | ◎ | ◎ | ○ | Δ |
| Extensibility on the skin | ◎ | ◎ | ◎ | ◎ | ◎ | ○ | Δ |

(Results)

From the results in Table 9, it can be seen that when an amount of incorporation of a polymer which forms a gel having no fluidity and a melting point of 40˚C or higher is about 0.1 % or less by mass in an aqueous phase, the foam stability is inferior. In addition, it can be seen that, when the amount is about 13% or more by mass, whipping ability is deteriorated, and the usability is inferior. Therefore, it is provided that an amount of a polymer which forms a gel having no fluidity and a melting point of 40˚C or higher, to be incorporated, is suitably 0.1-13 % by mass, more suitably 1-7% by mass.

B-2 Amount of a nonionic surfactant. lecithin and casein to be incorporated

Whipped O/W type emulsion cosmetics having the formulations in Table 10 was prepared, an amount of a nonionic surfactant, lecithin and casein to be incorporated was studied.

Table 10

| Ingredient | Test Example | | | | | | |
|---|---|---|---|---|---|---|---|
| | 59 | 60 | 61 | 62 | 63 | 64 | 65 |
| Glyceryl monostearate | 0.02 | 0.06 | 0.25 | 1.0 | 5.0 | 7.0 | 9.0 |
| Casein | 0.02 | 0.06 | 0.25 | 1.0 | 5.0 | 7.0 | 9.0 |
| Glucomannan | 1.0 | 1.0 | 1.0 | 1.0 | 1.0 | 1.0 | 1.0 |
| Kertrol | 1.0 | 1.0 | 1.0 | 1.0 | 1.0 | 1.0 | 1.0 |

(continued)

| Ingredient | Test Example | | | | | | |
|---|---|---|---|---|---|---|---|
| | 59 | 60 | 61 | 62 | 63 | 64 | 65 |
| Dynamite glyceline | 5.0 | 5.0 | 5.0 | 5.0 | 5.0 | 5.0 | 5.0 |
| Phenoxyethanol | 0.2 | 0.2 | 0.2 | 0.2 | 0.2 | 0.2 | 0.2 |
| Purified water | Balance | | | | | | |
| EDTA | 0.05 | 0.05 | 0.05 | 0.05 | 0.05 | 0.05 | 0.05 |
| Stearyl alcohol | 8.0 | 8.0 | 8.0 | 8.0 | 8.0 | 8.0 | 8.0 |
| Squalane | 2.0 | 2.0 | 2.0 | 2.0 | 2.0 | 2.0 | 2.0 |
| Pentaeryththritol 2-hexanoate | 8.0 | 8.0 | 8.0 | 8.0 | 8.0 | 8.0 | 8.0 |
| Soybean lecithin | 0.02 | 0.02 | 0.02 | 0.1 | 0.5 | 1.0 | 1.0 |
| Amount of a nonionic surfactant, lecithin and casein, to be incorporated | | | | | | | |
| | 0.06 | 0.14 | 0.52 | 2.10 | 10.5 | 15.0 | 19.0 |
| < Overrun degree > | Δ | ○ | ◎ | ◎ | ◎ | ◎ | ◎ |
| < Foam stability > | | | | | | | |
| 37˚C Particle diameter of foam | Δ | ○ | ◎ | ◎ | ◎ | ◎ | ◎ |
| Disappearance of foam | Δ | ○ | ◎ | ◎ | ◎ | ◎ | ◎ |
| < Usability > | | | | | | | |
| Scoopability with a finger | Δ | ○ | ◎ | ◎ | ◎ | ◎ | ◎ |
| Extensibility on the skin | Δ | ○ | ◎ | ◎ | ◎ | ○ | Δ |

(Results)

From the results in Table 10, it can be discovered that, when an amount of a nonionic surfactant, lecithin and casein to be incorporated is less than 0.1% by mass, in sufficient whipping occurs, decreasing overrun degree and the foam stability and the usability are inferior. Moreover, when the amount is 15% or greater by mass, the usability is inferior. Therefore, it can be seen that an amount of a nonionic surfactant, lecithin and casein is suitably about 0.1- about 15% by mass, more suitably about 0.5 -about 10% by mass.

B-3 Amount of an oil to be incorporated

Whipped O/W type emulsion cosmetics having the formulations in Table 11 were prepared, and an amount of a solid oil to be incorporated at a temperature of 30˚C or lower was studied.

Table 11

| Ingredient | Test Example | | | | | |
|---|---|---|---|---|---|---|
| | 66 | 67 | 68 | 69 | 70 | 71 |
| Sucrose monostearate | 1.0 | 1.0 | 1.0 | 1.0 | 1.0 | 1.0 |
| Casein | 1.0 | 1.0 | 1.0 | 1.0 | 1.0 | 1.0 |
| Gellan gum | 2.0 | 2.0 | 2.0 | 2.0 | 2.0 | 2.0 |
| Calcium Chloride | 0.4 | 0.4 | 0.4 | 0.4 | 0.4 | 0.4 |
| 1,3-butyleneglycol | 5.0 | 5.0 | 5.0 | 5.0 | 5.0 | 5.0 |
| Methylparaben | 0.2 | 0.2 | 0.2 | 0.2 | 0.2 | 0.2 |
| Purified water | Balance | | | | | |
| Sodium hexametaphosphate | 0.05 | 0.05 | 0.05 | 0.05 | 0.05 | 0.05 |
| Liquid paraffin | 20.0 | 17.0 | 12.0 | 8.0 | 3.0 | - |
| Microcrystallinewax | 1.0 | 1.0 | 1.0 | 1.0 | 1.0 | 1.0 |
| Palm hydrogenated oil | - | 3.0 | 8.0 | 12.0 | 17.0 | 20.0 |
| Soybean lecithin | 0.1 | 0.1 | 0.1 | 0.1 | 0.1 | 0.1 |
| Tarc | 2.0 | 2.0 | 2.0 | 2.0 | 2.0 | 2.0 |
| Amount of a solid oil ingredient to be incorporated in whole oil ingredient | | | | | | |

(continued)

| Ingredient | | Test Example | | | | | |
|---|---|---|---|---|---|---|---|
| | | 66 | 67 | 68 | 69 | 70 | 71 |
| | | 0 | 15 | 40 | 60 | 85 | 100 |
| <Overrun degree> | | ◎ | ◎ | ◎ | ◎ | ○ | Δ |
| < Foam stability > | | | | | | | |
| 37˚C | Particle diameter of foam | × | Δ | ◎ | ◎ | ◎ | ◎ |
| | Disappearance of foam | Δ | Δ | ◎ | ◎ | ◎ | ◎ |
| <Usability> | | | | | | | |
| | Scoopability with a finger | ○ | ○ | ○ | ○ | ○ | ○ |
| | Extensibility on the skin | ◎ | ◎ | ◎ | ◎ | ○ | ○ |

(Results)

From the results in Table 11, it can be seen that, when an amount of solid oil to be incorporated in the whole oil is 20% or less by mass, overrun degree and foam stability are decreased. Therefore, an amount of a solid oil in the whole oil to be incorporated is suitably 20% by mass or more, more suitably 40% by mass or more.

Table 12

| Ingredient | | Test Example | | | | | | | |
|---|---|---|---|---|---|---|---|---|---|
| | | 72 | 73 | 74 | 75 | 76 | 77 | 78 | 79 |
| Palm hydrogenated oil | | 10.0 | 10.0 | 10.0 | 10.0 | 10.0 | - | 10.0 | - |
| Beeswax | | 10.0 | 10.0 | 10.0 | 10.0 | 10.0 | - | 10.0 | - |
| Liquid paraffin | | - | 1.0 | 1.0 | 1.0 | - | 20.0 | - | - |
| Sucrose monostearate | | 1.0 | 1.0 | 2.0 | 1.0 | 1.0 | 1.0 | 0.01 | 1.0 |
| Glyceryl monostearate | | - | - | - | - | - | 4.0 | - | - |
| POE hydrogenated castor oil | | - | 0.3 | - | - | - | - | - | - |
| Casein | | 1.0 | 1.0 | - | 1.0 | 1.0 | 1.0 | 0.01 | 1.0 |
| Soybean lecithin | | 0.1 | 0.1 | | 0.1 | 0.1 | 0.1 | 0.01 | 0.1 |
| Agar | | 2.0 | 2.0 | 1.0 | 2.0 | - | 2.0 | 2.0 | 2.0 |
| Gellan gum | | - | - | 1.0 | - | - | - | - | - |
| Xanthane gum | | - | 0.1 | - | - | 2.0 | - | - | - |
| 1,3-butyleneglycol | | 5.0 | 5.0 | 5.0 | 5.0 | 5.0 | 5.0 | 5.0 | 5.0 |
| Methylparaben | | 0.2 | 0.2 | 0.2 | 0.2 | 0.2 | 0.2 | 0.2 | 0.2 |
| Purified water | | Balance | | | | | | | |
| Sodium hexametaphosphate | | 0.05 | 0.05 | 0.05 | 0.05 | 0.05 | 0.05 | 0.05 | 0.05 |
| Polyethylene powder | | - | - | - | 2.0 | - | - | - | - |
| Sodium citrate | | - | - | 0.5 | - | - | - | - | - |
| < Overrun degree(%) > | | 100 | 100 | 60 | 80 | 100 | 5 | 5 | 140 |
| < Foam stability > | | | | | | | | | |
| 0˚C | Particle diameter of foam | ◎ | ◎ | ◎ | ◎ | ◎ | ◎ | ◎ | ◎ |
| | Disappearance of foam | ◎ | ◎ | ◎ | ◎ | ◎ | ◎ | ◎ | ◎ |
| 37˚C | Particle diameter of foam | ◎ | ◎ | ◎ | ◎ | × | × | Δ | ◎ |
| | Disappearance of foam | ◎ | ◎ | ◎ | ◎ | × | × | Δ | ◎ |
| < Usability > | | | | | | | | | |
| | Scoopability with a finger | ◎ | ◎ | ◎ | ◎ | ◎ | ◎ | Δ | × |
| | Extensibility on the skin | ◎ | ◎ | ◎ | ◎ | ◎ | ◎ | Δ | × |

(Results)

From the results in Table 12, it can be seen that the present whipped O/W type emulsion cosmetic has the better foam stability and the excellent usability.

Example 6 Moisture-retaining mask

| | |
|---|---|
| (1) Purified water | Balance |
| (2) Phenoxyethanol | 0.3 |
| (3) 1,3-butyleneglycol | 5.0 |
| (4) Ascorbic acid | 0.5 |
| (5) Glucomannan | 0.5 |
| (6) Xanthane gum | 1.0 |
| (7) Sucrose oleate | 1.0 |
| (8) Saponin | 0.5 |
| (9) Sodium caseinate | 0.5 |
| (10) Beeswax | 10.0 |
| (11) Vaseline | 15.0 |
| (12) Olefine oligomer | 5.0 |
| (13) Soybean lecithin | 0.1 |
| (14) Polyglyceryl stearate | 1.0 |
| (15) Nylon powder | 1.0 |
| (16) Perfume | q.s. |

(Process for preparation)

To an aqueous phase in which ingredients (1)-(9) were mixed and dissolved at 80˚C, were added (10)-(14) and (15) uniformly dissolved at 80˚C and (16), which was uniformly dispersed with a homomixer, cooled, whipped with a batch mixer at room temperature to prepare a whipped O/W type emulsion cosmetic, which was filled in a container at room temperature and cooled as it was. The resulting whipped cream has the better foam stability and the excellent usability.

Example 7 Moisture-retaining cream

| | |
|---|---|
| (1) Purified water | Balance |
| (2) Methylparaben | 0.2 |
| (3) Dipropyleneglycol | 5.0 |
| (4) Arbtin | 1.0 |
| (5) Treharose | 0.5 |
| (6) Sodium hexametaphosphate | |
| (7) Casein | 0.5 |
| (8) Calcium lactate | 0.1 |
| (9) Sucrose monooleate polyester | 1.0 |
| (10) Carageenan | 1.0 |
| (11) Gellan gum | 1.0 |
| (12) Stearyl alcohol | 3.0 |
| (13) Behenyl alcohol | 4.0 |
| (14) Isopropyl myristate | 4.0 |
| (15) Glyceryl monostearate | 2.0 |
| (16) Purified soybean lecithin | 0.08 |
| (17) Perfume | q.s. |

(Process for preparation)

To an aqueous phase in which ingredients (1)-(9) were mixed and dissolved at 80˚C, were added (12)-(17) uniformly dissolved at 80˚C, which was uniformly dispersed with a homomixer, and cooled to room temperature. This O/W phase was whipped with a batch mixer at about 40˚C, and (10) and (11) dissolved in a part of (1) at 80˚C were mixed in the whipped cream, which was filled in a container at about 50˚C and cooled as it was. The resulting whipped cream had the better foam stability and the excellent usability

Example 8 Massage cream

| | |
|---|---|
| (1) Purified water | Balance |
| (2) Phenoxyethanol | 0.3 |
| (3) Polyethyleneglycol | 5.0 |
| (4) Trymethylglycine | 0.5 |
| (5) Glyceryl monostearate | 1.0 |
| (6) Sorbitan oleate | 1.0 |
| (7) Sodium caseinate | 0.5 |
| (8) Glucomannnan | 0.5 |
| (9) Xanthane gum | 0.5 |
| (10) Saponin ,aqueous solution | 1.0 |
| (11) Microcrystalline wax | 10.0 |
| (12) Palm hydrogenated oil | 15.0 |
| (13) Macadamianut oil | 5.0 |
| (14) Soybean lecithin | 0.1 |
| (15) Hydrophobing starch powder | 1.0 |
| (16) Perfume | q.s. |

(Process for preparation)

To an aqueous phase in which ingredients (1)-(7) were mixed and dissolved at 80˚C, were added (11)-(16) uniformly dissolved at 80˚C, which was uniformly dispersed with a homomixer, mixed with (8)-(10) dissolved in a part of (1), which was whipped with a batch mixer at 30-40˚C to prepare a whipped O/W type emulsion cosmetic. The prepared whipped cream had the better foam stability and the excellent usability.

Example 9 Moisture-retaining cream

| | |
|---|---|
| (1) Purified water | Balance |
| (2) Methylparaben | 0.2 |
| (3) Propyleneglycol | 7.0 |
| (4) Sodium hexametaphosphate | 0.3 |
| (5) Glyceryl monostearate (self-emulsified type) | 0.5 |
| (6) POE(60)glyceryl stearate | 1.0 |
| (7) Stearyl alcohol | 2.0 |
| (8) Behenyl alcohol | 1.5 |
| (9) Alkylsilicone wax | 1.0 |
| (10) Vaseline | 5.0 |
| (11) Squalane | 8.0 |
| (12) Pentaerythrityl tetraoctanoate | 8.0 |
| (13) Glucomannan | 1.0 |
| (14) Kertrol | 1.0 |

(Process for preparation)

To an aqueous phase in which ingredients (1)-(5) were mixed and dissolved at 80˚C, were added (6)-(12) uniformly dissolved at 80˚C, which was uniformly dispersed with a homomixer. This was whipped with a batch mixer at 40˚C, mixed with (13) and (14) dissolved in a part of (1), which was filled in a container at 40˚C, and cooled to room temperature to prepare a whipped O/W type emulsion cosmetic. The prepared whipped cream had the better foam stability and the excellent usability.

Example 10 Moisture-retaining mask

| | |
|---|---|
| (1) Purified water | Balance |
| (2) Methylparaben | 0.2 |
| (3) Glycerin | 7.0 |
| (4) EDTA·3Na | 0.3 |
| (5) Glyceryl monostearate (self-emulsified type) | 1.0 |
| (6) POE glyceryl isostearate | 2.0 |
| (7) Stearyl alcohol | 2.0 |
| (8) Ceresin | 1.5 |
| (9) Beeswax | 7.0 |
| (10) Vaseline | 5.0 |
| (11) Olefine oligomer | 8.0 |
| (12) Glyceryl trioctanoate | 8.0 |
| (13) Hydrogenated soybean lecithin | 0.1 |
| (14) Agar | 1.0 |
| (15) Glucomannan | 0.3 |

(Process for preparation)

To an aqueous phase in which ingredients (1)-(5) were mixed and dissolved at 80˚C, were added (6)-(13) uniformly dissolved at 80˚C, which was uniformly dispersed with a homomixer. This was whipped with a batch mixer at 40˚C, mixed with (14) and (15) dissolved in a part of (1), which was filled in a container at 40˚C, and cooled to room temperature to prepare a whipped O/W type emulsion cosmetic. The prepared whipped cream had the better foam stability and the excellent usability.

**Claims**

1. A whipped O/W type emulsion cosmetic, which comprises a gel-foaming amount of a polymer which forms a gel having no fluidity, in an aqueous phase.

2. The whipped O/W type emulsion cosmetic according to claim 1, wherein the polymer which forms a gel having no fluidity is 1 or 2 or more kinds selected from gelatin, sodium alginate, carrageenan, gellan gum, glucomannan and curdlan.

3. The whipped O/W type emulsion cosmetic according to claim 1 or 2, wherein an amount of the polymer which forms a gel having no fluidity to be incorporated is 0.1-13% by mass in an aqueous phase.

4. The whipped O/W type emulsion cosmetic according to any one of claims 1-3, wherein said cosmetic contains a higher fatty acid soap.

5. The whipped O/W type emulsion cosmetic according to claim 4, wherein an amount of the higher fatty acid soap to be incorporated is 1-20% by mass in the whole cosmetic.

6. The whipped O/W type emulsion cosmetic according to any one of claims 1-5, wherein an amount of an oil to be incorporated is 1-30% by mass in the whole cosmetic.

7. The whipped O/W type emulsion cosmetic according to any one of claims 1-6, wherein said cosmetic contains a cationic polymer.

8. The whipped O/W type emulsion cosmetic according to claim 7, wherein an amount of the cationic polymer is 0.01-5% by mass in the whole cosmetic.

9. The whipped O/W type emulsion cosmetic according to claim 1 or 2, wherein a melting point of the polymer which forms a gel having no fluidity is 40°C or higher.

10. The whipped O/W type emulsion cosmetic according to claim 9, wherein said cosmetic contains a solid oil which is a solid at a normal temperature, and a nonionic surfactant as an emulsifier.

11. The whipped O/W type emulsion cosmetic according to claim 10, wherein an amount of the solid oil to be incorporated is 20-100% by mass in a total amount of the oil.

12. The whipped O/W type emulsion cosmetic according to any one of claims 9-11, wherein said cosmetic contains casein and/or lecithin.

13. The whipped O/W type emulsion cosmetic according to claim 12, wherein an amount of a nonionic surfactant, casein and lecithin to be incorporated is 0.1-15% by mass in the whole cosmetic.

14. A process for preparing a whipped O/W type emulsion cosmetic, which comprises whipping an O/W type emulsion containing a polymer which forms a gel having no fluidity, which is filled in the whipped state.

15. A process for preparing a whipped O/W type emulsion cosmetic, which comprises whipping an O/W type emulsion containing no polymer which forms a gel having no fluidity, then mixing a polymer which forms a gel having no fluidity, in a whipped base, and which is filled.


**Patentansprüche**

1. Geschlagene kosmetische O/W-Emulsion, umfassend in der wässrigen Phase eine gelbildende Menge eines Polymers, das ein nicht fließfähiges Gel bildet.

2. Geschlagene kosmetische O/W-Emulsion nach Anspruch 1, wobei das Polymer, das ein nicht fließfähiges Gel bildet, eine, zwei oder mehrere aus Gelatine, Natriumalginat, Carrageen, Gellangummi, Glucomannan und Curdlan ausgewählte Spezies ist/sind.

3. Geschlagene kosmetische O/W-Emulsion nach Anspruch 1 oder 2, wobei die Menge des zu inkorporierenden Polymers, das ein nicht fließfähiges Gel bildet, 0,1 bis 13 Massenprozent der wäßrigen Phase beträgt.

4. Geschlagene kosmetische O/W-Emulsion nach einem der Ansprüche 1 bis 3, wobei das Kosmetikum eine Seife einer höheren Fettsäure enthält.

5. Geschlagene kosmetische O/W-Emulsion nach Anspruch 4, wobei die Menge der zu inkorporierende Seife einer höheren Fettsäure 1 bis 20 Massenprozent des gesamten Kosmetikums beträgt.

6. Geschlagene kosmetische O/W-Emulsion nach einem der Ansprüche 1 bis 5, wobei die Menge des zu inkorporierenden Öls 1 bis 30 Masseprozent des gesamten Kosmetikums beträgt.

7. Geschlagene kosmetische O/W-Emulsion nach einem der Ansprüche 1 bis 6, wobei das Kosmetikum ein kationisches Polymer enthält.

8. Geschlagene kosmetische O/W-Emulsion nach Anspruch 7, wobei die Menge des kationischen Polymers 0,01 bis 5 Masseprozent des gesamten Kosmetikums beträgt.

**9.** Geschlagene kosmetische O/W-Emulsion nach Anspruch 1 oder 2, wobei der Schmelzpunkt des gelbildenden Polymers, das ein nicht fließfähiges Gel bildet, 40 ˚C oder höher ist.

**10.** Geschlagene kosmetische O/W-Emulsion nach Anspruch 9, wobei das Kosmetikum ein festes Öl, das bei normaler Temperatur fest ist, und als Emulgator ein nichtionisches Netzmittel enthält.

**11.** Geschlagene kosmetische O/W-Emulsion nach Anspruch 10, wobei die Menge des zu inkorporierenden festen Öls 20 bis 100 Massenprozent der Ölphase beträgt.

**12.** Geschlagene kosmetische O/W-Emulsion nach einem der Ansprüche 9 bis 11, wobei das Kosmetikum Casein und/ oder Lecithin enthält.

**13.** Geschlagene kosmetische O/W-Emulsion nach Anspruch 12, wobei die Menge des zu inkorporierenden nichtionischen Netzmittels, Caseins und Lecithins 0,1 bis 15 Masseprozent des gesamten Kosmetikums beträgt.

**14.** Verfahren zur Herstellung einer geschlagenen kosmetischen O/W-Emulsion, umfassend das Aufschlagen einer O/W-Emulsion, welche ein Polymer enthält, das ein nicht fließfähiges Gel bildet, die im geschlagenen Zustand gefüllt wird.

**15.** Verfahren zur Herstellung einer geschlagenen kosmetischen O/W-Emulsion, umfassend das Aufschlagen einer O/W-Emulsion, welche kein Polymer enthält, das ein nicht fließfähiges Gel bildet, danach Einmischen eines Polymers, das ein nicht fließfähiges Gel bildet, in die geschlagene Grundsubstanz, die dann gefüllt wird.

**Revendications**

**1.** Produit cosmétique en émulsion de type H/E fouettée, qui comprend une quantité gélifiante d'un polymère qui forme un gel n'ayant aucune fluidité dans une phase aqueuse.

**2.** Produit cosmétique en émulsion de type H/E fouettée selon la revendication 1, dans lequel le polymère qui forme un gel n'ayant aucune fluidité est de 1 ou 2 types ou plus choisis parmi la gélatine, l'alginate de sodium, la carraghénine, la gomme gellane, le glucomannane et le curdlane.

**3.** Produit cosmétique en émulsion de type H/E fouettée selon la revendication 1 ou 2, dans lequel la quantité du polymère qui forme un gel n'ayant aucune fluidité à incorporer est de 0,1 à 13 % en masse dans une phase aqueuse.

**4.** Produit cosmétique en émulsion de type H/E fouettée selon l'une quelconque des revendications 1 à 3, dans lequel ledit produit cosmétique contient un savon à base d'acides gras supérieurs.

**5.** Produit cosmétique en émulsion de type H/E fouettée selon la revendication 4, dans lequel la quantité de savon à base d'acides gras supérieurs à incorporer est de 1 à 20 % en masse dans le produit cosmétique global.

**6.** Produit cosmétique en émulsion de type H/E fouettée selon l'une quelconque des revendications 1 à 5, dans lequel la quantité d'une huile à incorporer est de 1 à 30 % en masse dans le produit cosmétique global.

**7.** Produit cosmétique en émulsion de type H/E fouettée selon l'une quelconque des revendications 1 à 6, dans lequel ledit produit cosmétique contient un polymère cationique.

**8.** Produit cosmétique en émulsion de type H/E fouettée selon la revendication 7, dans lequel la quantité de polymère cationique est de 0,01 à 5 % en masse dans le produit cosmétique global.

**9.** Produit cosmétique en émulsion de type H/E fouettée selon la revendication 1 ou 2, dans lequel le point de fusion du polymère qui forme un gel n'ayant aucune fluidité est de 40 ˚C ou supérieur.

**10.** Produit cosmétique en émulsion de type H/E fouettée selon la revendication 9, dans lequel ledit produit cosmétique contient une huile solide qui est une matière solide à une température normale ainsi qu'un tensioactif non ionique comme émulsifiant.

11. Produit cosmétique en émulsion de type H/E fouettée selon la revendication 10, dans lequel la quantité d'huile solide à incorporer est de 20 à 100 % en masse dans la quantité totale d'huile.

12. Produit cosmétique en émulsion de type H/E fouettée selon l'une quelconque des revendications 9 à 11, dans lequel ledit produit cosmétique contient de la caséine et/ou de la lécithine.

13. Produit cosmétique en émulsion de type H/E fouettée selon la revendication 12, dans lequel la quantité de tensioactif non ionique, de caséine et de lécithine à incorporer est de 0,1 à 15 % en masse dans le produit cosmétique global.

14. Procédé pour préparer un produit cosmétique en émulsion de type H/E fouettée qui comprend le fouettement d'une émulsion de type H/E contenant un polymère qui forme un gel n'ayant aucune fluidité qui est versée à l'état fouetté.

15. Procédé pour préparer un produit cosmétique en émulsion de type H/E fouettée qui comprend le fouettement d'une émulsion de type H/E ne contenant pas de polymère qui forme un gel n'ayant aucune fluidité, puis le mélange d'un polymère qui forme un gel n'ayant aucune fluidité, dans une base fouettée, et qui est versée.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000262977 A
- JP 2000262978 A
- JP 56079613 A
- JP 9249522 A
- JP 11209262 A